# EUROPÄISCHE PATENTSCHRIFT

(11) **EP 4 140 284 B1**
(45) Veröffentlichungstag und Bekanntmachung des Hinweises auf die Patenterteilung: **29.07.2026**
(21) Anmeldenummer: 22173928.7
(22) Anmeldetag: 18.05.2022
(51) Int. Cl.: A01D 43/08, G01N 15/06, G01N 27/22, G01N 33/00, G01F 1/64, G01N 15/01

(54) **FELDHÄCKSLER MIT KAPAZITIVER SENSORIK ZUR FEUCHTE-, DICHTE- UND DURCHSATZMESSUNG**
FORAGE HARVESTER COMPRISING CAPACITIVE SENSOR SYSTEM FOR MEASURING MOISTURE, DENSITY AND THROUGHPUT
SYSTÈME DE DÉTECTION CAPACITIVE POUR LA MESURE DE L'HUMIDITÉ, DE LA DENSITÉ ET DU DÉBIT

(30) Priorität: 26.08.2021 DE 102021122079; 31.08.2021 DE 102021122455
(43) Veröffentlichungstag der Anmeldung: 01.03.2023
(73) Patentinhaber: CLAAS Selbstfahrende Erntemaschinen GmbH, 33428 Harsewinkel (DE)
(72) Erfinder: Lehmann, Christoph, 49191 Belm (DE); Neitemeier, Dennis, 59510 Lippetal (DE); Heitmann, Christoph, 48231 Warendorf (DE)
(74) Vertreter: CLAAS Gruppe

(56) Entgegenhaltungen:
- DE-A1- 102005 017 121
- DE-A1- 102020 204 601
- DE-A1- 4 318 477
- DE-A1- 4 442 711

## Beschreibung

Die vorliegende Erfindung betrifft einen selbstfahrenden Feldhäcksler und ein Verfahren zum Steuern dieses Feldhäckslers.

Typischerweise wird in einem selbstfahrenden Feldhäcksler der Durchsatz von Erntegut über die Einzugswalzenhöhe bestimmt, wobei dieses Messsystem regelmäßig kalibriert werden muss, da wesentliche Einflussgrößen über die Einzugswalzenhöhe nicht erfasst werden können. Zu diesen wesentlichen Einflussgrößen zählen beispielsweise die Materialeigenschaften, insbesondere die Feuchtigkeit, des Ernteguts. Eine Feuchtigkeitsmessung wird in selbstfahrenden Feldhäcksler entweder mit einem Leitfähigkeitssensor oder mit einem Nahinfrarot-Sensor bzw. NIR-Sensor realisiert. Eine Feuchtigkeitsmessung mit einem Leitfähigkeitssensor ist aus der DE 10 2007 053 910 A1 bekannt. Die Fehler bei der Leitfähigkeitsmessung mit einem Leitfähigkeitssensor sind relativ groß, da wesentliche Einflussgrößen nur unzureichend kompensiert werden können. Ein NIR-Sensor führt im Vergleich zum Leitfähigkeitssensor zu qualitativ besseren Feuchtigkeitsmessungen, weist jedoch im Vergleich zu einem Leitfähigkeitssensor signifikant höhere Herstellungskosten auf.

Die DE 10 2005 017121 A1 offenbart einen Feldhäcksler mit einem Erntegutsensor im Auswurfkrümmer, wobei zur Verbesserung der Detektionsfähigkeit verschiedene Krümmerquerschnitte vorgeschlagen werden.

Die DE 10 2020 204601 A1 schlägt zur Erfassung von Ernteguteigenschaften wie dem Testgewicht einer Erntegutprobe einen Dielektrizitätskonstante-Sensor mit planar zueinander angeordneten Elektroden vor.

Die DE 44 42 711 A1 und die DE 43 18 477 A1 offenbaren jeweils kapazitive Sensorvorrichtungen zur Erfassung von Materialeigenschaften eines Volumenstroms, bspw. von Erntegut.

Der vorliegenden Erfindung liegt die Aufgabe zugrunde, eine verbesserte oder zumindest eine alternative Ausführungsform eines selbstfahrenden Feldhäckslers mit einer Messvorrichtung anzugeben, wobei insbesondere eine Verringerung der Herstellungskosten der Messvorrichtung bereitgestellt wird.

Diese Aufgabe wird erfindungsgemäß durch die Gegenstände der unabhängigen Ansprüche gelöst. Vorteilhafte Ausführungsformen sind Gegenstand der abhängigen Ansprüche.

Die vorliegende Erfindung beruht auf dem allgemeinen Gedanken, dass eine Messvorrichtung mehrere voneinander beabstandete Elektroden aufweist, die im Auswurfkrümmer angeordnet sind, wobei diese im Auswurfkrümmer angeordneten Elektroden mehrere Kondensatoren ausbilden.

Der erfindungsgemäße selbstfahrende Feldhäcksler zur Aufnahme und Behandlung von Erntegut weist einen Auswurfkrümmer zum Abtransport des Ernteguts und die Messvorrichtung zur Erfassung förderspezifischer Parameter und/oder gutspezifischer Parameter auf. Die Kapazität der Kondensatoren im Auswurfkrümmer wird durch zerkleinertes Erntegut bzw. Häckselgut, welches von Häckseltrommeln und/oder einer Konditioniereinrichtung gehäckselt wurde, beeinflusst. Häckselgut weist im Vergleich zu Luft eine signifikant höhere Permittivität auf. Die höhere Permittivität des Häckselguts kann an einem höheren Wasseranteil im Vergleich zur Luft liegen. Diese Eigenschaft führt dazu, dass sich die Kapazität eines Kondensators verändert, wenn das Häckselgut in Vergleich zu Luft in der Nähe des Kondensators ist.

Förderspezifische Parameter und/oder gutspezifische Parameter können die Feuchtigkeit des geförderten Erntegutes und/oder die Dichte des geförderten Erntegutes und/oder die Durchsatzmenge des geförderten Erntegutes und/oder die Querschnittsverteilung des geförderten Erntegutes sein. Die Querschnittsverteilung des geförderten Erntegutes kann von der Führung des Erntegutes im Vorsatzgerät und/oder von der Führung des Erntegutes im Erntegutbearbeitungskanal und/oder von Prozessparametern und/oder von Pflanzenparametern abhängig sein. Die Kapazität eines Kondensators wird maßgeblich von der Feuchtigkeit bzw. Gutfeuchte, also einer Veränderung der Permittivität des Materials, der Dichte bzw. Verdichtung und der Durchsatzmenge bzw. der Gutmenge beeinflusst. Durch Messungen der elektrischen Kapazitäten der mehreren Kondensatoren sind die förderspezifischen Parameter und/oder gutspezifischen Parameter ermittelbar.

Der selbstfahrende Feldhäcksler weist somit eine kapazitive Sensorik zur Feuchtemessung des Häckselgut und/oder zur Dichtemessung des Häckselgut und/oder zur Durchsatzmessung des Häckselgut und/oder zur Messung der Querschnittsverteilung des Häckselguts auf. Die Messvorrichtung kann wenigstens vier voneinander beabstandet angeordnete Elektroden aufweisen.

Die Elektroden können jeweils eine Schutzeinrichtung, insbesondere eine elektrisch schwachleitende Schicht und/oder eine nichtleitende Schicht, aufweisen. Diese Schutzeinrichtung kann aus einem nicht-metallischen Material ausgebildet sein. Die Elektroden können für eine berührungslose Messung innerhalb wenigstens einer Wandung des Auswurfkrümmers und/oder wenigstens an einer Wandung des Auswurfkrümmers angeordnet sein.

Der Vorteil der Messvorrichtung mit mehreren Kondensatoren ist, dass die Kosten für die Herstellung der Messvorrichtung geringer sind als die Kosten für die Herstellung einer Messvorrichtung mit einem NIR-Sensor. Ein weiterer Vorteil ist darin zu sehen, dass die Messvorrichtung mit mehreren Kondensatoren im Vergleich zu einer Messvorrichtung mit einem Leitfähigkeitssensor messgenauer ist. Hierdurch kann ein selbstfahrender Feldhäcksler mit einer messgenaueren Messvorrichtung und/oder günstigeren Messvorrichtung zur Messung von förderspezifischer Parameter und/oder gutspezifischer Parameter ausgestattet werden.

Bei einer vorteilhaften Weiterbildung der erfindungsgemäßen Lösung ist vorgesehen, dass die Messvorrichtung dazu ausgebildet ist, mittels der gemessenen Kapazitäten der Kondensatoren eine Feuchtigkeitsmessung zur Ermittlung der Feuchtigkeit des Ernteguts auszuführen. Die Kapazität der Kondensatoren wird von der Feuchtigkeit bzw. Gutfeuchte des zerkleinerten Ernteguts bzw. Häckselguts, welches das elektrische Feld der Kondensatoren durchströmt, beeinflusst. Bei einer Messung der Kapazität der Kondensatoren hat der Trockensubstanzgehalt (TS) des Häckselguts einen messbaren Einfluss auf die Kapazität, sodass in Abhängigkeit der Kapazität der Kondensatoren der Trockensubstanzgehalt (TS) des Häckselguts oder die Feuchtigkeit des Häckselguts ermittelbar ist. Es kann vorgesehen sein, dass diese Messungen beispielsweise mittels einer LC-Schwingkreismethode und/oder mittels einem Wechselspannungsmessverfahren ausgeführt werden. Bei einer Verwendung eines Wechselspannungsmessverfahren werden geringere Fehler in der Echtzeit generiert.

Bei einer vorteilhaften Weiterbildung der erfindungsgemäßen Lösung ist vorgesehen, dass die Messvorrichtung dazu ausgebildet ist, mittels der gemessenen Kapazitäten der Kondensatoren eine Durchsatzmessung zur Ermittlung des Durchsatzes des Ernteguts auszuführen. Der Durchsatz kann beispielsweise dadurch ermittelt werden, dass der Trockensubstanzgehalt (TS) für eine bestimmte Zeit, beispielsweise 1 Sekunde, ermittelt wird und der Durchsatz dann dem Trockensubstanzgehalt (TS) pro Sekunde entspricht. Es können auch andere Zeitbereiche und/oder Parameter zu Bestimmung des Durchsatzes festgelegt sein.

Eine Beeinflussung der Kapazität von Kondensatoren hängt von der Beabstandung der Elektroden ab. Hat ein erster Kondensator beispielsweise zwei voneinander beabstandete Elektroden, deren Beabstandung größer ist als die Beabstandung von zwei voneinander beabstandete Elektroden eines zweiten Kondensators, dann ist die Kapazität des ersten Kondensators vom Durchsatz abhängig ist, während die Kapazität des zweiten Kondensators stärker von der Feuchtigkeit abhängig ist.

Bei einer vorteilhaften Weiterbildung der erfindungsgemäßen Lösung ist vorgesehen, dass die Messvorrichtung dazu ausgebildet ist, mittels der gemessenen Kapazitäten der Kondensatoren eine Dichtemessung zur Ermittlung der Dichte des Ernteguts auszuführen. Bei einer Messung der Kapazität der Kondensatoren hat eine Ernteguthöhe des Häckselguts einen messbaren Einfluss auf die Kapazität. Die Dichte des Ernteguts kann beispielsweise dadurch ermittelt werden, dass die Ernteguthöhe des Häckselguts für alle Kondensatoren ermittelt wird. Die Dichte des Ernteguts kann sich beispielweise aus dem Trockensubstanzgehalt (TS) geteilt durch die Ernteguthöhe des Häckselguts ergeben. Es können auch andere Definitionen der Dichte des Ernteguts festgelegt sein, die ermittelt werden können. Beispielsweise ist die Dichte des Ernteguts nach einem Aufbereitungsaggregat abhängig von der Aufbereitung des Ernteguts durch eine Konditioniereinrichtung bzw. einen Corn-Cracker. Bei einer konstanten Häcksellänge kann aus dem Signal der Dichte eine Veränderung der Aufbereitung erfasst werden und das Aufbereitungsaggregat gegebenenfalls nachgesteuert werden. Die Messung einer Größe, die direkt von der Aufbereitung abhängig ist, kann eine Grundlage für eine anschließende automatische Steuerung sein.

Bei einer vorteilhaften Weiterbildung der erfindungsgemäßen Lösung ist vorgesehen, die Messvorrichtung dazu ausgebildet ist, mittels der gemessenen Kapazitäten aller Kondensatoren eine Querverteilung des Ernteguts zu ermitteln. Insbesondere lässt sich mittels der gemessenen Kapazitäten aller Kondensatoren eine Querverteilung des Ernteguts ermitteln und eine Optimierung und/oder Verbesserung der Querverteilung des Ernteguts im Falle einer nicht gleichmäßigen Querverteilung des Ernteguts bewirken.

Bei einer vorteilhaften Weiterbildung der erfindungsgemäßen Lösung ist vorgesehen, dass jeder Kondensator genau zwei Elektroden aufweist, wobei diese Elektroden nicht von einem anderen Kondensator nutzbar sind. Mit anderen Worten ausgedrückt, ist zu jeder Elektrode stets lediglich eine Gegenelektrode vorhanden. Wenn beispielsweise zwei Kondensatoren vorhanden sind, sind genau vier Elektroden vorhanden, wobei jeder Kondensator durch zwei Elektroden ausgebildet ist. Mit einem solchen Aufbau ist beispielsweise eine differentielle Messung möglich, bei der die Kapazität stets zwischen zwei Elektroden gemessen wird.

Bei einer vorteilhaften Weiterbildung der erfindungsgemäßen Lösung ist vorgesehen, dass alle Kondensatoren jeweils eine Elektrode aufweisen, wobei diese Elektroden jeweils mit einer gemeinsamen Elektrode, insbesondere mit einer gemeinsamen Sendeelektrode, nutzbar sind. Mit anderen Worten ausgedrückt, teilen sich alle Kondensatoren die gemeinsame Elektrode bzw. die gemeinsame Sendeelektrode. Dies hat den Vorteil, dass bei einer Anzahl von N Kondensatoren lediglich N+1 Elektroden erforderlich sind. Daher können die Kosten und auch der erforderliche Einbauraum für die Kondensatoren deutlich reduziert werden. Außerdem kann diese Ausgestaltung aus Bauraumgründen vorheilhaft sein, da eine deutlich größere Anzahl an unterschiedlichen Abständen der Elektroden der Kondensatoren auf einer Fläche ausbildbar ist.

Erfindungsgemäß ist vorgesehen, dass die Messvorrichtung mehrere Elektroden aufweist, die unterschiedliche Beabstandungen zueinander und/oder unterschiedliche Beabstandungen zu einer gemeinsamen Elektrode aufweisen. Insbesondere können wenigstens vier Elektroden eine unterschiedliche Beabstandungen zueinander und/oder wenigstens drei Elektroden eine unterschiedliche Beabstandungen zu einer gemeinsamen Elektrode aufweisen. Die Beabstandungen zwischen den Elektroden können parallel und/oder quer zur Förderrichtung des Erntegutstromes ausgebildet sein. Je größer der Abstand zwischen den Elektroden eines Kondensators ist, desto größer ist die Feldeindringung des elektrischen Feldes des Kondensators in das am Kondensator vorbeiströmende Erntegut. Durch die unterschiedliche Beabstandung der Elektroden können unterschiedliche Eindringtiefen des elektrischen Feldes erzielt werden, sodass die Feuchtigkeit und der Durchsatz voneinander getrennt messbar sind.

Die Feuchtigkeit des Ernteguts und/oder der Durchsatz des Ernteguts und/oder Dichte des Ernteguts und/oder die Querverteilung des Ernteguts haben einen unterschiedlich starken Einfluss auf die Kapazität der Kondensatoren bzw. auf ein Sensorsignal der Kondensatoren bei unterschiedlichen Abständen der Elektroden.

Bei einer vorteilhaften Weiterbildung der erfindungsgemäßen Lösung ist vorgesehen, dass die Messvorrichtung mehrere Elektroden aufweist, die unterschiedliche geometrische Ausgestaltungen aufweisen. Es kann beispielweise vorgesehen sein, dass wenigstens drei Elektroden und/oder drei Elektrodenarten der Messvorrichtung unterschiedliche geometrische Ausgestaltungen aufweisen.

Beispielsweise kann die gemeinsame Elektrode, insbesondere die gemeinsame Sendeelektrode, einen Längsabschnitt aufweisen, der sich entlang einer Längsrichtung erstreckt. Außerdem kann die gemeinsame Elektrode, insbesondere die gemeinsame Sendeelektrode, zusätzlich mehrere Querabschnitte aufweisen, die sich jeweils entlang einer Querrichtung erstrecken. Hierbei ist die Längsrichtung quer und/oder senkrecht zur Querrichtung ausgerichtet. Der Längsabschnitt und die mehreren Querabschnitte der gemeinsamen Elektrode, insbesondere der gemeinsamen Sendeelektrode, bilden gemeinsam ein Bauteil, insbesondere ein einteiliges Bauteil, aus. Die gemeinsame Elektrode kann wenigstens zwei Querabschnitte aufweisen. Die Querabschnitte der gemeinsamen Sendeelektrode können bezüglich der Längsrichtung voneinander beabstandet, insbesondere äquidistant voneinander beabstandet, angeordnet sein. Die Längsrichtung kann quer oder parallel zur Förderrichtung des Erntegutstromes ausgereichtet sein.

Eine Elektrode, die nicht die gemeinsame Elektrode ist, kann eine Nahelektrode ausbilden. Die Nahelektrode kann einen Längsabschnitt aufweisen, der sich entlang der Längsrichtung erstreckt. Der Längsabschnitt der Nahelektrode kann parallel zum Längsabschnitt der gemeinsamen Elektrode ausgerichtet und/oder beabstandet zur gemeinsamen Elektrode angeordnet sein. Die Nahelektrode kann zusätzlich mehrere Querabschnitte aufweisen, die sich jeweils entlang der Querrichtung erstrecken. Der Längsabschnitt und die mehreren Querabschnitte der Nahelektrode bilden gemeinsam ein Bauteil, insbesondere ein einteiliges Bauteil, aus. Die Querabschnitte der Nahelektrode können bezüglich der Längsrichtung voneinander beabstandet, insbesondere äquidistant voneinander beabstandet, angeordnet sein. Die Querabschnitte der gemeinsamen Elektrode und die Querabschnitte der Nahelektrode können bezüglich der Längsrichtung wenigstens abschnittsweise gegenüberliegend und/oder voneinander beabstandet angeordnet sein. Die Nahelektrode kann wenigstens zwei Querabschnitte aufweisen.

Eine Elektrode, die nicht die gemeinsame Elektrode und nicht die Nahelektrode ist, kann eine Mittelelektrode ausbilden. Die Mittelelektrode kann einen Längsabschnitt aufweisen, der sich entlang der Längsrichtung erstreckt. Der Längsabschnitt der Mittelelektrode kann parallel zum Längsabschnitt der gemeinsamen Elektrode ausgerichtet und/oder beabstandet zur gemeinsamen Elektrode angeordnet sein. Der Längsabschnitt der Mittelelektrode kann parallel zum Längsabschnitt der Nahelektrode ausgerichtet und/oder beabstandet zur Nahelektrode angeordnet sein. Die Nahelektrode kann, insbesondere bezüglich der Querrichtung, zwischen der gemeinsamen Elektrode und der Mittelelektrode angeordnet sein. Die Mittelelektrode kann bezüglich der Querrichtung eine Querrichtungsbreite aufweisen, die größer ist als die Querrichtungsbreite des Längsabschnitts der gemeinsamen Elektrode und/oder als die Querrichtungsbreite des Längsabschnitts der Nahelektrode.

Eine Elektrode, die nicht die gemeinsame Elektrode und nicht die Nahelektrode und nicht die Mittelelektrode ist, kann eine Außenelektrode ausbilden. Die Außenelektrode kann einen Längsabschnitt aufweisen, der sich entlang der Längsrichtung erstreckt. Der Längsabschnitt der Außenelektrode kann parallel zum Längsabschnitt der gemeinsamen Elektrode ausgerichtet und/oder beabstandet zur gemeinsamen Elektrode angeordnet sein. Der Längsabschnitt der Außenelektrode kann parallel zum Längsabschnitt der Nahelektrode ausgerichtet und/oder beabstandet zur Nahelektrode angeordnet sein. Der Längsabschnitt der Außenelektrode kann parallel zum Längsabschnitt der Mittelelektrode ausgerichtet und/oder beabstandet zur Mittelelektrode angeordnet sein. Die Außenelektrode kann bezüglich der Querrichtung eine Querrichtungsbreite aufweisen, die größer ist als die Querrichtungsbreite des Längsabschnitts der gemeinsamen Elektrode und/oder als die Querrichtungsbreite des Längsabschnitts der Nahelektrode. Die Nahelektrode und/oder die Mittelelektrode kann, insbesondere bezüglich der Querrichtung, zwischen der gemeinsamen Elektrode und der Außenelektrode angeordnet sein. Die Außenelektrode kann bezüglich der Querrichtung eine Querrichtungsbreite aufweisen, die der Querrichtungsbreite der Mittelelektrode gleicht.

Die Elektroden der Messvorrichtung können bezüglich Längsrichtung gespiegelt ausgebildet sein, wobei in diesem Fall der Schwerpunkt, insbesondere der Flächenschwerpunkt, der gemeinsamen Elektrode auf der Längsrichtung aufliegt. Somit sind beispielsweise sechs Elektroden ausgebildet, wobei lediglich eine Elektrode eine gemeinsame Elektrode ausbildet, während die Nahelektrode und die Mittelelektrode und die Außenelektrode jeweils zweifach ausgebildet sind. Diese gespiegelte Ausführung kann zu einer internen Validierung der Messergebnisse genutzt werden. Ferner können weitere Informationen, wie eine Verteilungserfassung, im Auswurfkrümmer ermittelt werden.

Bei einer vorteilhaften Weiterbildung der erfindungsgemäßen Lösung ist vorgesehen, dass alle Elektroden der Messvorrichtung in einer Ebene angeordnet sind. Mit anderen Worten ausgedrückt, kann hierdurch eine planare Messvorrichtung ausgebildet werden. Hierbei kann es vorgesehen sein, dass die Elektroden in einer Oberseite des Auswurfkrümmers angeordnet sind. Beispielsweise wird das Erntegut durch den Aufbau des Auswurfkrümmers an der Oberseite des Auswurfkrümmers angepresst. Daher wird das Erntegut beim Transport durch den Auswurfkrümmer an die Elektroden der Messvorrichtung im Wesentlichen angepresst, sodass eine Beabstandung zwischen den Elektroden und dem Erntegut vermieden wird. Hierdurch können signifikante Auswirkungen auf die Messwerte, insbesondere auf die Messwerte der Dichte des Erntegut, vermieden werden.

Bei einer vorteilhaften Weiterbildung der erfindungsgemäßen Lösung ist vorgesehen, dass einige, insbesondere alle, Elektroden der Messvorrichtung an einer Oberseite des Auswurfkrümmers und/oder in einer Oberseite des Auswurfkrümmers angeordnet sind. Die Elektroden können teilweise und/oder vollständig in der Oberseite des Auswurfkrümmers angeordnet sein. Eine vollständige Anordnung der Elektroden in der Oberseite des Auswurfkrümmers hat den Vorteil, dass die Elektroden keinen Durchströmungswiederstand für das Erntegut ausbilden. Daher ist der Transportleistung des Erntegutes im Auswurfkrümmer optimal.

Bei einer vorteilhaften Weiterbildung der erfindungsgemäßen Lösung ist vorgesehen, dass die Messvorrichtung wenigstens einen Wechselspannungserzeuger zum Erzeugen einer Wechselspannung aufweist, und/oder dass die Messvorrichtung wenigstens eine Verstärkereinheit zum Verstärken eines Signals aufweist, und/oder dass die Messvorrichtung wenigstens einen elektrischen Verstärker zur Umwandlung eines Eingangsstroms in eine proportionale Ausgangsspannung aufweist, und/oder dass die Messvorrichtung wenigstens einen Detektor, insbesondere wenigstens einen logarithmischen Detektor, zur Umwandlung eines einer Wechselspannung in ein Leitungssignal aufweist, und/oder dass die Messvorrichtung wenigstens einen Analog-Digital-Wandler zur Umwandlung eines analogen Signals in ein digitales, insbesondere digital interpretierbares, Signal aufweist.

Der Wechselspannungserzeuger kann eine Direct-Digital-Synthesis-Einheit sein. Die Verstärkereinheit kann ein eingehendes Signal mit einem festgelegten Faktor und/oder einstellbaren Faktor erhöht werden. Der elektrische Verstärker kann ein Transimpedanzverstärker sein, der einen Eingangsstrom in eine proportionale Ausgangsspannung umwandelt. Der logarithmische Detektor kann zur Wandlung der Wechselspannung eines Transimpedanzverstärkers in ein Leistungssignal ausgebildet sein. Die Messvorrichtung kann einen Anschlussbauteil oder mehrere Anschlussbauteile aufweisen. Ein Anschlussbauteil kann ein Eingangsbauteil oder ein Ausgangsbauteil sein. An einem solchen Anschlussbauteil können weitere Komponenten, insbesondere Sensoren, angeschlossen werden.

Hierbei kann der Wechselspannungserzeuger elektrisch leitend mit der Verstärkereinheit verbunden sein, wobei die Verstärkereinheit über ein Anschlussbauteil, welches ein Ausgangsbauteil ausbildet, mit einer ersten Elektrode eines Kondensators elektrisch leitend verbunden sein kann. Eine zweite Elektrode des Kondensators kann beabstandet zur ersten Elektrode angeordnet sein. Eine elektrische Stromleitung zwischen der ersten Elektrode und der zweiten Elektrode ist unterdrückt bzw. bildet sich nicht aus. Die zweite Elektrode des Kondensators kann mit einem weiteren Anschlussbauteil, welches ein Eingangsbauteil ausbildet, elektrisch leitend verbunden sein. Das weitere Anschlussbauteil kann mit dem Transimpedanzverstärker elektrisch leitend verbunden sein. Der Transimpedanzverstärker kann elektrisch leitend mit dem Detektor, insbesondere dem logarithmischen Detektor, verbunden sein. Der Detektor, insbesondere der logarithmische Detektor, kann elektrisch leitend mit der Verstärkereinheit verbunden sein. Die Verstärkereinheit kann elektrisch leitend mit dem Analog-Digital-Wandler verbunden sein. Der Analog-Digital-Wandler kann mit einem weiteren Anschlussbauteil, welches ein Ausgangsbauteil ausbildet, verbunden sein.

Bei einer vorteilhaften Weiterbildung der erfindungsgemäßen Lösung ist vorgesehen, dass die Messvorrichtung eine Steuerungs- und Regelungseinrichtung aufweist, die dazu ausgebildet ist, die Kapazität der Kondensatoren zu erfassen, wobei die Steuerungs- und Regelungseinrichtung dazu ausgebildet ist, mittels der erfassten Kapazitäten die förderspezifischen Parameter und/oder gutspezifischen Parameter des Ernteguts zu ermitteln. Die Steuerungs- und Regelungseinrichtung kann dazu ausgebildet sein, mittels der erfassten Kapazitäten der Kondensatoren die Feuchtigkeit des Ernteguts und/oder den Durchsatz des Ernteguts und/oder die Dichte des Ernteguts und/oder die Querverteilung des Ernteguts zu ermitteln. Ferner ist die Steuerungs- und Regelungseinrichtung dazu ausgebildet, Aktoren des Vorsatzgerätes und/oder Aktoren des Erntegutbearbeitungskanals anhand der ermittelten förderspezifischen Parameter und/oder gutspezifischen Parameter des Ernteguts zu steuern.

Die Steuerungs- und Regelungseinrichtung kann dazu ausgebildet sein, die Kapazitäten aller Kondensatoren simultan und/oder zeitgleich zu erfassen.

Die Steuerungs- und Regelungseinrichtung kann dazu ausgebildet sein, die Kapazitäten aller Kondensatoren nacheinander, insbesondere zeitlich nacheinander, zu erfassen. Mit anderen Worten ausgedrückt, wird hierbei die Kapazität zwischen zwei Elektroden ermittelt und zeitlich daran anschließend die Kapazität zwischen zwei weiteren Elektroden ermittelt.

In der Steuerungs- und Regelungseinrichtung können für jeden Kondensator mehrere Kennlinien hinterlegt und/oder gespeichert sein. Eine Kennlinie für einen Kondensator kann beispielsweise die Kapazität des Kondensators in Abhängigkeit von der Ernteguthöhe des Häckselguts ausbilden. In praktischen Messungen werden solche Kennlinien in Abhängigkeit der Ernteguthöhe und der Feuchtigkeit bzw. Gutfeuchte aufgezeichnet und/oder ermittelt. Je nach Abstand der Elektroden und/oder Geometrie der Elektroden haben die kapazitiven Sensoren unterschiedliche Kennlinien. Die Kennlinien sind in unterschiedlichem Maße abhängig von den förderspezifischen Parametern und/oder gutspezifischen Parametern. Diese Eigenschaft wird genutzt, um aus den Messwerten mittels einem Kennlinienfeld, also mehreren Kennlinien eines Kondensators, die gewünschten Informationen bzw. förderspezifischen Parameter und/oder gutspezifischen Parameter des Ernteguts zu extrahieren bzw. zu ermitteln.

Ferner betrifft die Erfindung ein Verfahren gemäß Anspruch 13.

Die ermittelten förderspezifischen Parameter und/oder gutspezifischen Parameter können beispielsweise die Feuchtigkeit des Ernteguts und/oder der Durchsatz des Ernteguts und/oder die Dichte des Ernteguts und/oder die Querverteilung des Ernteguts sein. Es kann ein Ansteuern der Aktoren des Vorsatzgerätes und/oder Aktoren des Erntegutbearbeitungskanals in Abhängigkeit von den ermittelten förderspezifischen Parametern und/oder gutspezifischen Parametern des Ernteguts vorgesehen sein. Die ermittelten förderspezifischen Parameter und/oder gutspezifischen Parametern des Ernteguts können beispielsweise an einen Kunden übermittelt werden. Die ermittelten förderspezifischen Parameter und/oder gutspezifischen Parametern des Ernteguts können zur optimalen Einstellung von Bauteilen des selbstfahrenden Feldhäckslers und/oder aller Bauteile des selbstfahrenden Feldhäckslers eingesetzt werden.

Die Erfassung einer Kapazität kann einer Messung der Kapazität entsprechen. Die Erfassung der Kapazität kann mittels unterschiedlicher Verfahren ausgeführt werden. Hierfür kann ein Lade-/Entladeverfahren, ein Oszillationsverfahren und/oder ein Wechselspannungsansatz eingesetzt werden. Die Erfassung der Kapazität kann im bzw. mit einem Gleichfeld oder Wechselfeld erfolgen. Hierbei kann das Wechselfeld eine Frequenz aus dem niederfrequenten Spektrum, insbesondere bis zu 30 kHz oder bis zu 3 kHz oder bis 300 Hz oder bis zu 30 Hz, eingesetzt werden. Die Messung kann seriell oder parallel durchgeführt werden. Dies bedeutet, dass die Messungen der einzelnen Kapazitäten gleichzeitig oder zeitlich nacheinander durchgeführt werden kann.

Die Erfassung und Digitalisierung der Kapazitäten kann vorzugsweise mit einem Wechselspannungsverfahren realisiert werden. Weitere mögliche Schaltungen sind beispielsweise ein RC-Schwingkreis, LC-Schwingkreis oder eine Lade-/Entladeschaltung. Der Vorteil in der Wechselspannungsmessung ist die hohe Immunität gegen Streukapazitäten und der hohen möglichen Abtastraten. Auch kann durch die Verwendung des Wechselspannungsverfahrens die Dimension der Frequenz der Wechselspannung mit in die Messung einbezogen werden. Dies hat den Vorteil, dass eine Kompensation des Feuchtigkeitseinflusses aus den Messergebnissen möglich ist. Die Frequenz hat durch den unterschiedlichen Einfluss der Leitfähigkeit des Häckselgutes Einfluss auf das Sensorsignal. Dies hat den Vorteil, dass der Sensor über den gesamten nötigen Messbereich die Feuchtigkeit und den Durchsatz bestimmen kann. Das Sensorergebnis kann durch die Hinzunahme weiterer Informationen, wie der Schichthöhe oder eines Feuchtesensors, verbessert werden, da so eine bessere Abschätzung einer Einflussgröße getroffen werden kann. Für die Bestimmung des Durchsatzes kann die Information der Gutgeschwindigkeit für die Verbesserung des Messergebnisses hilfreich sein. Dies führt dazu, dass der Durchsatz auch bei unterschiedlichen Drehzahlen der Aggregate noch kalibriert werden können.

Bei einer vorteilhaften Weiterbildung der erfindungsgemäßen Lösung ist vorgesehen, dass das Erfassen der Kapazität der Kondensatoren zeitlich nacheinander und/oder dass das Erfassen der Kapazität der Kondensatoren zeitgleich erfolgt.

Eine zeitlich nacheinander ablaufende Messung der Kapazitäten kann als serieller Messablauf bezeichnet werden. Beim seriellen Messablauf wird ein einziges Elektrodenpaar ausgewertet und die Kapazität des Elektrodenpaars bestimmt. Hierbei können die Kapazitäten aller Kondensatoren nacheinander, insbesondere zeitlich nacheinander, erfasst werden. Nach einer Messung wird das nächste Elektrodenpaar angesteuert und ausgewertet. Um eine verbesserte Auflösung der Querverteilung des zerkleinerten Ernteguts bzw. Häckselguts zu ermöglichen, kann vorgesehen sein, dass nicht nur bezüglich der Querrichtung gegenüberliegende Elektroden ausgewertet werden, sondern auch weiter entfernte Elektroden. Durch eine zuvor bestimmte Sensitivitätsverteilungsmatrix kann durch diese zusätzlichen Messungen die Querverteilungsberechnung verbessert werden. Nach dem Durchlauf der Messungen können die Messergebnisse unter Hinzunahme der Sensitivitätsverteilungsmatrix ausgewertet und/oder eine Permittivitätsverteilung bezüglich der Querrichtung ermittelt bzw. berechnet werden. Diese Permittivitätsverteilung kann als Steuergröße für die Optimierung der Querverteilung des zerkleinerten Ernteguts bzw. Häckselguts eingesetzt werden. Die Permittivitätsverteilung kann als Steuergröße für das Ansteuern der Aktoren des Vorsatzgerätes und/oder der Aktoren des Einzugskanals eingesetzt werden.

Eine zeitlich parallel ablaufende Messung der Kapazitäten kann beispielsweise mit einer einzelnen Gegenelektrode bzw. Sendeelektrode erfolgen. Die einzige Gegenelektrode kann dabei mit der Vielzahl von Elektroden jeweils einen Kondensator ausbilden, wobei die Kapazitäten dieser Kondensatoren zeitgleich erfassbar bzw. messbar sind. Hierbei kann ein Wechselspannungsansatz verwendet werden. Auf die Gegenelektrode kann ein harmonisches Signal angelegt werden. Auf einer Erfassungsseite können die Kapazitäten kontinuierlich ausgewertet werden. Dies hat den Vorteil, dass die Fehler durch zeitliche Änderungen des Durchsatzes minimiert werden. Durch die Erfassung der Querverteilung kann das Durchsatzsignal des Feldhäckslers optimiert werden. Dies ermöglicht, da durch die ermittelte Verteilung beispielsweise Ungleichmäßigkeiten in einer Gutmatte kompensiert werden können.

Bei einer vorteilhaften Weiterbildung der erfindungsgemäßen Lösung ist vorgesehen, dass eine Optimierung der Querverteilung des Ernteguts im Falle einer nicht gleichmäßigen Querverteilung des Ernteguts initiiert wird, wobei hierfür das Ansteuern der Aktoren des Vorsatzgerätes und/oder der Aktoren des Einzugskanals in Abhängigkeit von der ermittelten Querverteilung des Ernteguts erfolgt. Hierdurch kann eine Optimierung und/oder Verbesserung der Querverteilung des Ernteguts im Falle einer nicht gleichmäßigen Querverteilung des Ernteguts bewirkt werden.

Ferner betrifft die Erfindung ein Computerprogramm, insbesondere ein Computerprogrammprodukt, umfassend Befehle, die bewirken, dass die erfindungsgemäße Vorrichtung die erfindungsgemäßen Verfahrensschritte ausführt.

Ferner betrifft die Erfindung ein computerlesbares Medium, auf dem das erfindungsgemäße Computerprogramm ist.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung nähererläutert, wobei sich gleiche Bezugszeichen auf gleiche oder ähnliche oder funktional gleiche Komponenten beziehen
Es zeigen, jeweils schematisch,
- Fig. 1: einen erfindungsgemäßen selbstfahrenden Feldhäcksler, und
- Fig. 2: eine erfindungsgemäße Elektrodenanordnung, und
- Fig. 3: die erfindungsgemäße Elektrodenanordnung in einer anderen Ansicht, und
- Fig. 4: eine erfindungsgemäße Messschaltung zur Ermittlung von förderspezifischen Parameter und/oder gutspezifischen Parameter des Ernteguts, und
- Fig.5: mehrere Kapazitätenverläufe der erfindungsgemäßen Kondensatoren in Abhängigkeit der Ernteguthöhe, und
- Fig. 6: ein Kapazitätsverlauf eines erfindungsgemäßen Kondensators in Abhängigkeit von der Ernteguthöhe und dem Trockensubstanzgehalt, und
- Fig. 7: ein Kapazitätsverlauf eines weiteren erfindungsgemäßen Kondensators in Abhängigkeit von der Ernteguthöhe und dem Trockensubstanzgehalt, und
- Fig. 8: ein Kapazitätsverlauf eines weiteren erfindungsgemäßen Kondensators in Abhängigkeit von der Ernteguthöhe und dem Trockensubstanzgehalt.

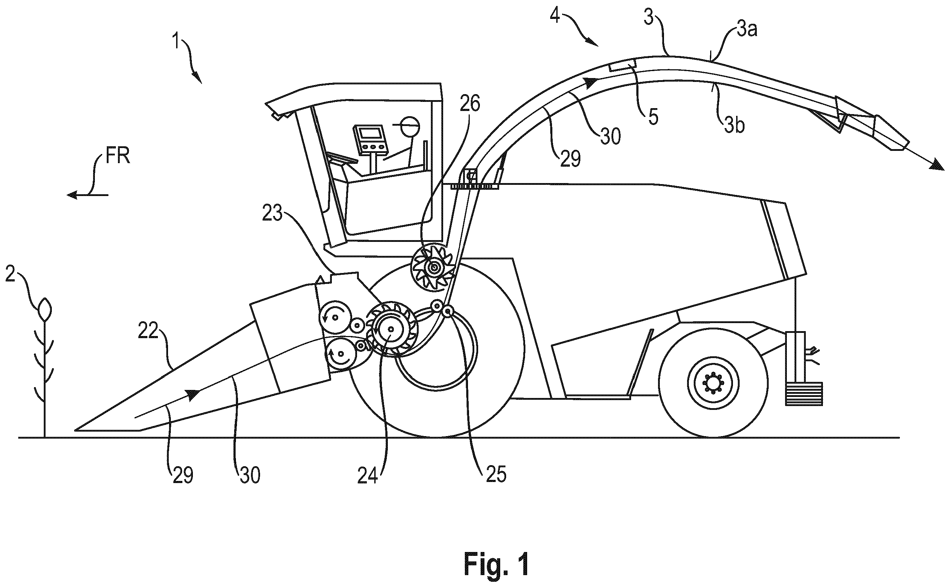

Die Fig. 1 zeigt einen selbstfahrenden Feldhäcksler 1. Der Feldhäcksler 1 weist ein Vorsatzgerät 22 zur Aufnahme von Erntegut 2 auf. Im Betrieb des Feldhäckslers 1 fährt der Feldhäcksler 1 in eine Fahrtrichtung FR durch einen Erntegutbestand 2 und nimmt dabei mit dem Vorsatzgerät 22 das Erntegut 2 auf. Im Erntebetrieb des Feldhäckslers 1 wird das Erntegut 2 vom Vorsatzgerät 22 geschnitten. Das geschnittene Erntegut 2 bildet einen Erntegutstrom 30 aus, der entlang einer Förderrichtung 29 gefördert wird.

Das Vorsatzgerät 22 ist austauschbar am Feldhäcksler 1 angeordnet. In Abhängigkeit vom Erntegut 2 und dem Erntegutzweck können so verschiedene Vorsatzgeräte 22 genutzt werden. Als Vorsatzgeräte 22 kommen beispielsweise eine Pick up, ein Maisvorsatz, ein Mähwerk (Direct Disc), ein Maispflücker oder ein Schneidwerk, insbesondere mit Haspel, zum Einsatz.

Das Vorsatzgerät 22 nimmt das Erntegut 2 auf und fördert es zu einem Erntegutbearbeitungskanal 23 des Feldhäcksler 1, wobei der Erntegutbearbeitungskanal 23 das Erntegut 2 bearbeitet und anschließend zu einem Auswurfkrümmer 3 des Feldhäckslers 1 fördert. Der Erntegutbearbeitungskanal 23 ist dem Vorsatzgerät 22 bezüglich der Förderrichtung 29 nachgeordnet.

Der Erntegutbearbeitungskanal 23 weist eine Häckseltrommel 24 zum Verarbeiten des Ernteguts 2 und eine Nachbeschleunigungstrommel 26 zum Nachbeschleunigen des Ernteguts 2 auf. Der Erntegutbearbeitungskanal 23 kann eine Konditioniereinrichtung 25 zur Konditionierung des Ernteguts 2 aufweisen. Die Konditioniereinrichtung 25 ist bezüglich der Förderrichtung 29 zwischen der Häckseltrommel 24 und der Nachbeschleunigungstrommel 26 angeordnet.

Der Feldhäcksler 1 weist eine Messvorrichtung 4 zur Erfassung förderspezifischer Parameter und/oder gutspezifischer Parameter auf. Diese Messvorrichtung 4 weist mehrere voneinander beabstandete Elektroden 5 auf, die im Auswurfkrümmer 3 angeordnet sind. Diese im Auswurfkrümmer 3 angeordneten Elektroden 5 bilden mehrere Kondensatoren 20 aus. Durch Messungen der elektrischen Kapazitäten der mehreren Kondensatoren 20 können die förderspezifischen Parameter und/oder gutspezifischen Parameter ermittelt werden.

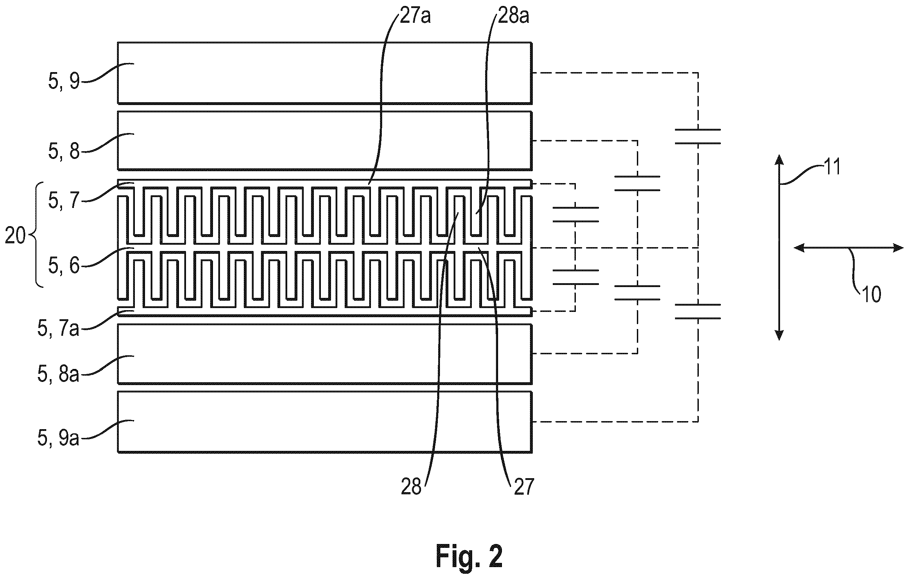

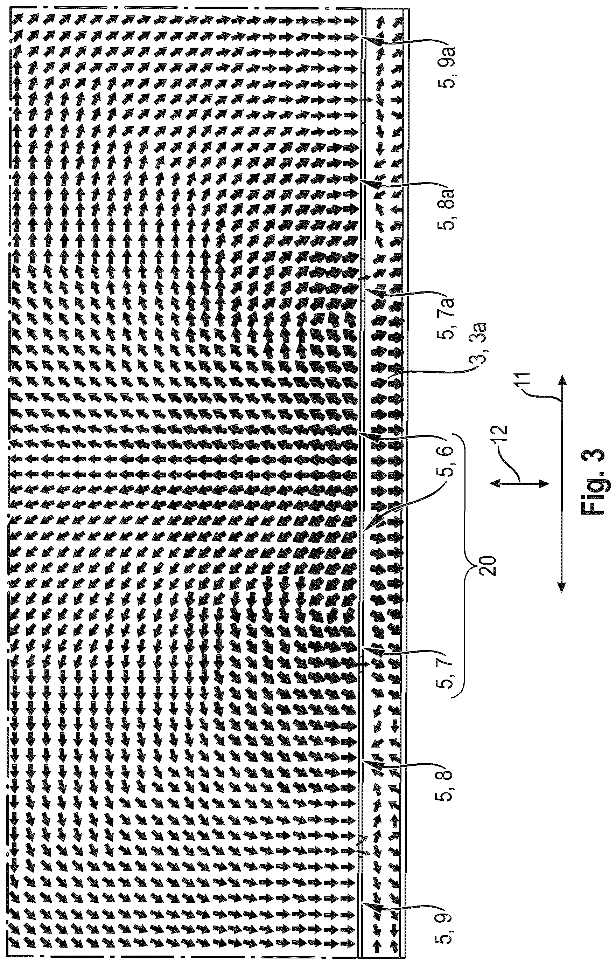

Wie aus der Fig. 1 hervorgeht, sind die Elektroden 5 der Messvorrichtung 4 an einer Oberseite 3a des Auswurfkrümmers 3 und/oder in einer Oberseite 3a des Auswurfkrümmers 3 angeordnet. Die Oberseite 3a kann einen größeren Krümmungsradius als eine Unterseite 3b des Auswurfkrümmers 3 aufweisen. Die Anordnung der Elektroden 5 der Messvorrichtung 4 ist beispielhaft in den Fig. 2 und Fig. 3 dargestellt.

Die Fig. 2 zeigt mehrere Elektroden 5, die wenigstens teilweise unterschiedliche geometrische Ausgestaltungen aufweisen. Es kann beispielsweise vorgesehen sein, dass wenigstens drei Elektroden 5 der Messvorrichtung 4 unterschiedliche geometrische Ausgestaltungen aufweisen.

In der Fig. 2 ist eine Ausgestaltung dargestellt, bei der die Elektroden 7, 8, 9, 7a, 8a, 9a mit einer gemeinsamen Elektrode 6, die eine gemeinsamen Sendeelektrode 6 ist, beispielsweise sechs Kondensatoren 20 ausbilden. Mit anderen Worten ausgedrückt, teilen sich alle Kondensatoren 7, 8, 9, 7a, 8a, 9a die gemeinsame Elektrode 6 bzw. die gemeinsame Sendeelektrode 6. Daher bildet die Elektrode 7 mit der gemeinsamen Sendeelektrode 6 einen ersten Kondensator 20. Die Elektrode 8 bildet mit der gemeinsamen Sendeelektrode 6 einen zweiten Kondensator. Die Elektrode 9 bildet mit der gemeinsamen Sendeelektrode 6 einen dritten Kondensator. Die Elektrode 7a bildet mit der gemeinsamen Sendeelektrode 6 einen vierten Kondensator. Die Elektrode 8a bildet mit der gemeinsamen Sendeelektrode 6 einen fünften Kondensator. Die Elektrode 9a bildet mit der gemeinsamen Sendeelektrode 6 einen sechsten Kondensator. Dies hat den Vorteil, dass bei einer Anzahl von sechs Kondensatoren lediglich sieben Elektroden erforderlich sind.

Die gemeinsame Sendeelektrode 6 weist einen Längsabschnitt 27 auf, der sich entlang einer Längsrichtung 10 erstreckt. Außerdem weist die gemeinsame Sendeelektrode 6 mehrere Querabschnitte 28 auf, die sich jeweils entlang einer Querrichtung 11 erstrecken. Hierbei ist die Längsrichtung 10 quer und/oder senkrecht zur Querrichtung 11 ausgerichtet. Der Längsabschnitt 10 und die mehreren Querabschnitte 11 der gemeinsamen Sendeelektrode 6 bilden gemeinsam ein Bauteil, insbesondere ein einteiliges Bauteil, aus. Die gemeinsame Sendeelektrode 6 kann wenigstens zwei Querabschnitte 11 aufweisen. Die Längsrichtung 10 kann quer oder parallel zur Förderrichtung 29 des Erntegutstromes 20 im Auswurfkrümmer 3 ausgereichtet sein.

Die Elektrode 5, die nicht die gemeinsame Sendeelektrode 6 ist, bildet eine Nahelektrode 7 aus. Die Nahelektrode 7 weist einen Längsabschnitt 27a auf, der sich entlang der Längsrichtung 10 erstreckt. Der Längsabschnitt 27a der Nahelektrode 7 ist parallel zum Längsabschnitt 27 der gemeinsamen Sendeelektrode 6 ausgerichtet und beabstandet zur gemeinsamen Sendeelektrode 6 angeordnet. Die Nahelektrode 7 kann zusätzlich mehrere Querabschnitte 28a aufweisen, die sich jeweils entlang der Querrichtung 11 erstrecken. Der Längsabschnitt 27a und die mehreren Querabschnitte 28a der Nahelektrode 7 bilden gemeinsam ein Bauteil, insbesondere ein einteiliges Bauteil, aus. Die Querabschnitte 28 der gemeinsamen Sendeelektrode 6 und die Querabschnitte 28a der Nahelektrode 7 sind bezüglich der Längsrichtung 10 wenigstens abschnittsweise gegenüberliegend und beabstandet zueinander angeordnet. Die Nahelektrode 7 kann wenigstens zwei Querabschnitte 27a aufweisen.

Die Elektrode 5, die nicht die gemeinsame Sendeelektrode 6 und nicht die Nahelektrode 7 ist, bildet eine Mittelelektrode 8 aus. Die Mittelelektrode 8 kann einen Längsabschnitt aufweisen, der sich entlang der Längsrichtung 10 erstreckt. Der Längsabschnitt der Mittelelektrode 8 ist parallel zum Längsabschnitt 27 der gemeinsamen Sendeelektrode 6 ausgerichtet und beabstandet zur gemeinsame Sendeelektrode 6 angeordnet. Der Längsabschnitt der Mittelelektrode 8 ist parallel zum Längsabschnitt 27a der Nahelektrode 7 ausgerichtet und beabstandet zur Nahelektrode 7 angeordnet. Die Nahelektrode 7 ist, insbesondere bezüglich der Querrichtung 11, zwischen der gemeinsamen Sendeelektrode 6 und der Mittelelektrode 8 angeordnet. Die Mittelelektrode 8 weist bezüglich der Querrichtung 11 eine Querrichtungsbreite auf, die größer ist als die Querrichtungsbreite des Längsabschnitts 27 der gemeinsamen Sendeelektrode 6 und/oder als die Querrichtungsbreite des Längsabschnitts 27a der Nahelektrode 7.

Die Elektrode 5, die nicht die gemeinsame Sendeelektrode 6 und nicht die Nahelektrode 7 und nicht die Mittelelektrode 8 ist, bildet eine Außenelektrode 9 aus. Die Außenelektrode 9 weist einen Längsabschnitt auf, der sich entlang der Längsrichtung 10 erstreckt. Der Längsabschnitt der Außenelektrode 9 ist parallel zum Längsabschnitt 27 der gemeinsamen Sendeelektrode 6 ausgerichtet und beabstandet zur gemeinsamen Sendeelektrode 6 angeordnet. Der Längsabschnitt der Außenelektrode 9 ist parallel zum Längsabschnitt 27a der Nahelektrode 7 ausgerichtet und beabstandet zur Nahelektrode 7 angeordnet. Der Längsabschnitt der Außenelektrode 9 ist parallel zum Längsabschnitt der Mittelelektrode 8 ausgerichtet und beabstandet zur Mittelelektrode 8 angeordnet sein. Die Nahelektrode 7 und die Mittelelektrode 8 sind, insbesondere bezüglich der Querrichtung, zwischen der gemeinsamen Elektrode 6 und der Außenelektrode 9 angeordnet. Die Außenelektrode 9 weist bezüglich der Querrichtung 11 eine Querrichtungsbreite auf, die größer ist als die Querrichtungsbreite des Längsabschnitts 27 der gemeinsamen Sendeelektrode 6 und/oder als die Querrichtungsbreite des Längsabschnitts 27a der Nahelektrode 7. Die Außenelektrode 9 weist bezüglich der Querrichtung 11 eine Querrichtungsbreite auf, die der Querrichtungsbreite der Mittelelektrode 8 gleicht.

Die Elektroden 6, 7, 8, 9 der Messvorrichtung können bezüglich Längsrichtung 10 gespiegelt ausgebildet sein, wobei in diesem Fall der Schwerpunkt, insbesondere Flächenschwerpunkt, der gemeinsame Elektroden 6 auf der Längsrichtung 10 aufliegt. Somit ergeben sich die Elektroden 7a, 8a, 9a. Hierbei sind die Elektroden 7 und 7a vom Aufbau gleichartig ausgebildet. Außerdem sind die Elektroden 8, 9, 8a, 9a vom Aufbau gleichartig ausgebildet.

Die Fig. 3 zeigt die Anordnung der Elektroden 5 in einer Ebene, die von der Querrichtung 11 und einer Höhenrichtung 12 ausgebildet ist. Die Höhenrichtung 12 ist quer zur Querrichtung 11 und quer zur Längsrichtung 10 ausgerichtet. In der Fig. 3 ist beispielhaft das elektrische Feld zwischen den Elektroden 5 angedeutet. Das Erntegut 2, dass durch diese Felder strömt, beeinflusst die Kapazität der Kondensatoren 20. Es ist gut erkennbar, dass eine Feldeindringung des elektrischen Feldes eines Kondensators 20 in das nicht dargestellte Erntegut 2 desto größer ist, wenn der Abstand zwischen den Elektroden 5 größer ist.

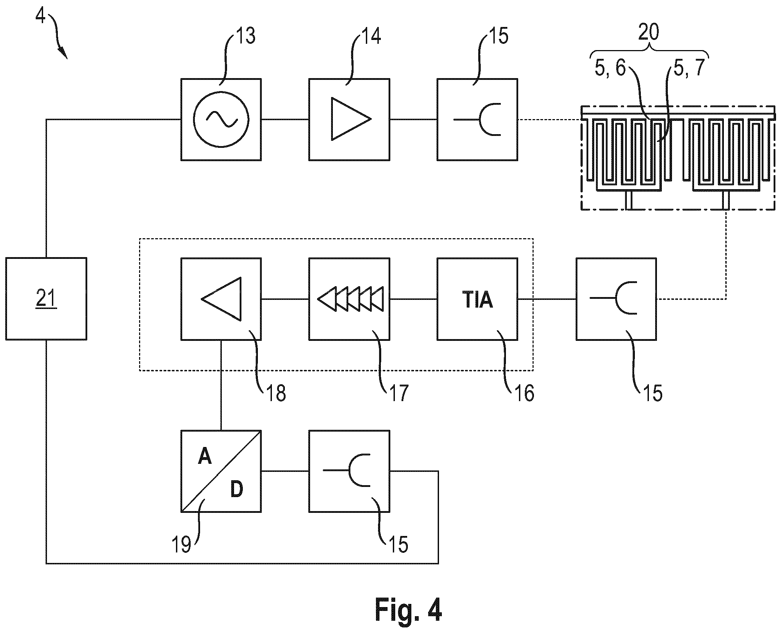

Gemäß der Fig. 4 weist die Messvorrichtung 4 wenigstens einen Wechselspannungserzeuger 13 zum Erzeugen einer Wechselspannung, eine Verstärkereinheit 14 zum Verstärken eines Signals, einen elektrischen Verstärker 16 zur Umwandlung eines Eingangsstroms in eine proportionale Ausgangsspannung, einen Detektor 17, insbesondere wenigstens einen logarithmischen Detektor, zur Umwandlung eines einer Wechselspannung in ein Leitungssignal, eine weitere Verstärkereinheit 18 zum Verstärken eines Signals, und wenigstens einen Analog-Digital-Wandler 19 zur Umwandlung eines analogen Signals in ein digitales, insbesondere digital interpretierbares, Signal auf.

Der Wechselspannungserzeuger 13 ist elektrisch leitend mit der Verstärkereinheit 14 verbunden, wobei die Verstärkereinheit 14 über ein Anschlussbauteil 15, welches ein Ausgangsbauteil ausbildet, mit einer ersten Elektrode eines Kondensators 20 elektrisch leitend verbunden ist. Hierbei wird ein Eingangssignal für den Kondensator 20 in Form von Wechselspannung an die gemeinsame Sendeelektrode 5,6 eines Kondensators 20 übertragen. Eine zweite Elektrode 5,7 des Kondensators 20 ist beabstandet zur gemeinsame Sendeelektrode 5,6 angeordnet. Eine elektrische Stromleitung zwischen der gemeinsame Sendeelektrode 5,6 und der zweiten zweite Elektrode 5,7 des Kondensators 20 ist unterdrückt bzw. bildet sich nicht aus. Die zweite Elektrode 5,7 des Kondensators 20 kann mit einem weiteren Anschlussbauteil 15, welches ein Eingangsbauteil ausbildet, elektrisch leitend verbunden sein kann. Hierbei wird ein Ausgangssignal des Kondensators 20 an das Anschlussbauteil 15 übertragen. Das weitere Anschlussbauteil 15 kann mit dem Transimpedanzverstärker 16 elektrisch leitend verbunden sein. Der Transimpedanzverstärker 16 kann elektrisch leitend mit dem Detektor 17, insbesondere dem logarithmischen Detektor, verbunden sein. Der Detektor 17, insbesondere der logarithmische Detektor, kann elektrisch leitend mit der Verstärkereinheit 18 verbunden sein. Die Verstärkereinheit 18 kann elektrisch leitend mit dem Analog-Digital-Wandler 19 verbunden sein. Der Analog-Digital-Wandler 19 kann mit einem weiteren Anschlussbauteil, welches ein Ausgangsbauteil ausbildet, verbunden sein. Die Steuerungs- und Regelungseinrichtung 21 kann zur Steuerung des Wechselspannungserzeuger 13 sowie zum Auslesen bzw. erfassen der Kapazitäten der Kondensatoren 20 ausgebildet sein.

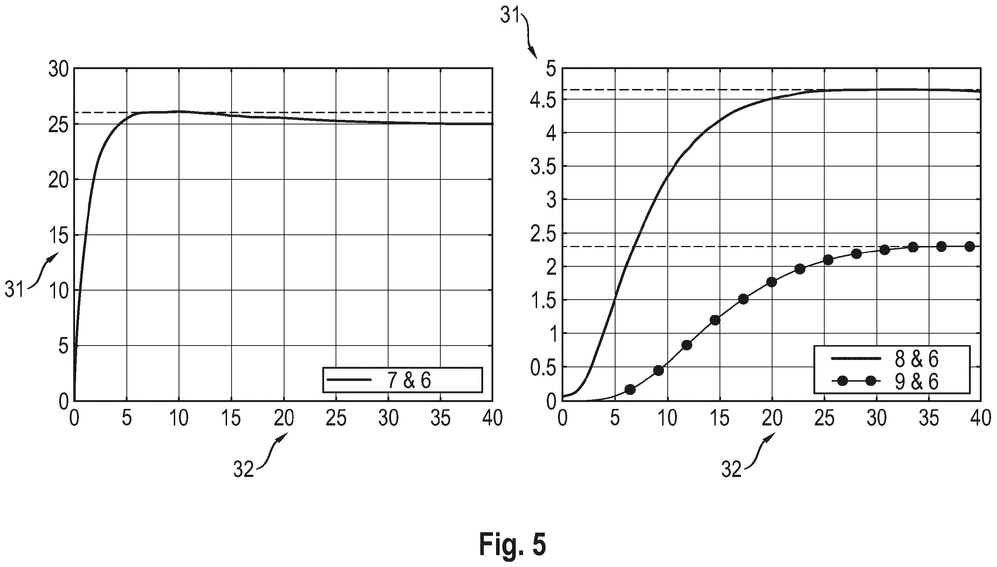

Für die dargestellte Struktur in der Fig. 2 ergeben sich unterschiedliche Verläufe der Kapazität in Abhängigkeit der Gutshöhe des Erntegutes 2 im Auswurfkrümmer 3. Die Gutshöhe kann beispielsweise bis zu 40 mm betragen. Beispielsweise ist in der Fig. 5, Fig. 6, Fig. 7 und Fig. 8 die Kapazität 31 mit der Einheit *"pF"* gegenüber der Ernteguthöhe bzw. Gutshöhe 32 mit der Einheit "*mm*" dargestellt. Hierbei ist beispielsweise der Kapazitätsverlauf für den Kondensator dargestellt, der durch die Sendeelektrode 6 und die Nahelektrode 7 ausgebildet ist, wobei dies in der Fig. 2 durch "*7&6*" angedeutet ist. Dieser Kondensator stellt beispielsweise die interdigitale Struktur des Sensors bzw. Messvorrichtung dar. Ferner ist der Kapazitätsverlauf für den Kondensator dargestellt, der durch die 6 Sendeelektrode und die Mittelelektrode 8 ausgebildet ist, wobei dies in der Fig. 2 durch "*8&6*" angedeutet ist. Ferner ist der Kapazitätsverlauf für den Kondensator dargestellt, der durch die 6 Sendeelektrode und die Außenelektrode 9 ausgebildet ist, wobei dies in der Fig. 2 durch "*9&6*" angedeutet ist. Hierbei sind unterschiedliche Verläufe der Kapazität 31 in Abhängigkeit der Erntegutshöhe 32 des Erntegutes 2 durch die verschiedenen Abstände und Geometrien der Elektroden 5 der Fig. 2 erkennbar. Die Änderung der Gutshöhe bei der niedrigen Beabstandung der Elektroden, z.B. bei 6 und 7 oder 6 und 7a, zeigt nur sehr minimale Veränderungen auf, sodass der Einfluss der Feuchte und der Dichte für diese Kondensatoren 20 deutlich signifikanter ist. Erkennbar ist beispielsweise, dass bei der niedrigen Beabstandung der Elektroden, z.B. bei 6 und 7 oder 6 und 7a, die Kapazität 31 ab ungefähr einer Gutshöhe 32 von 5 mm nur noch minimale Veränderungen aufweist.

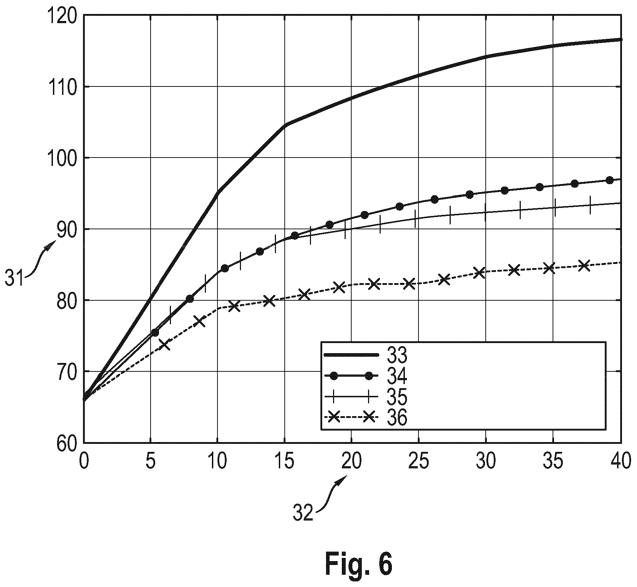

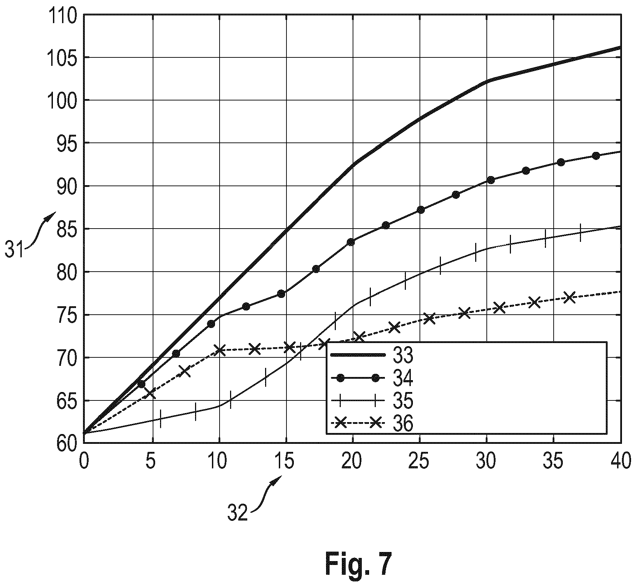

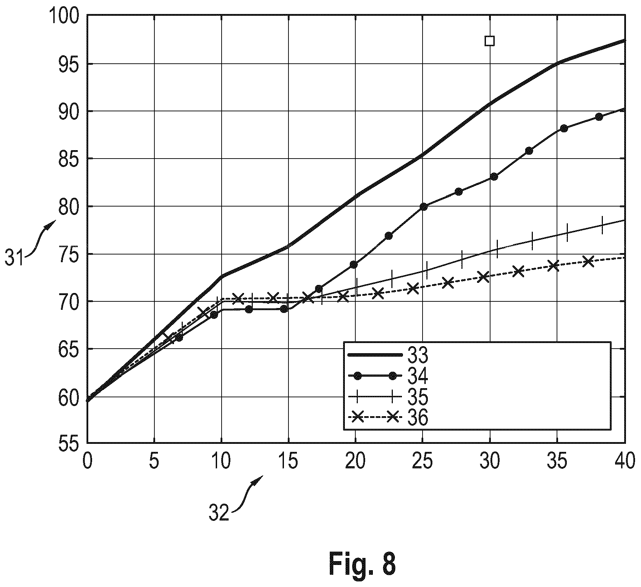

In den Fig. 6, Fig. 7 und Fig. 8 ist die Kapazität 31 in Abhängigkeit der Ernteguthöhe Gutshöhe 32 für verschiedene Gutsfeuchten bzw. Feuchtigkeiten des Ernteguts 2 bzw. Trockensubstanzgehälter des Ernteguts 2 bei einer konstanten Permittivität des Ernteguts 2 dargestellt. In den Fig. 6, Fig. 7 und Fig. 8 sind Kapazitäten unterschiedlicher eines Kondensatoren 20 gezeigt, wobei die Messwerte 33 bei einem Trockensubstanzgehalt von 64,50 % gemessen wurden, wobei die Messwerte 34 bei einem Trockensubstanzgehalt von 67,35 % gemessen wurden, wobei die Messwerte 35 bei einem Trockensubstanzgehalt von 70,22 % gemessen wurden und wobei die Messwerte 36 bei einem Trockensubstanzgehalt von 73,88 % gemessen wurden. Der deutliche Einfluss des Trockensubstanzgehaltes (TS) ist ersichtlich. Außerdem ist auch dieser Messwerteverlauf für unterschiedliche Kondensatoren 20 der Fig. 2 unterschiedlich. Mit anderen Worten ausgedrückt, wird die Kapazität 30 der unterschiedlichen Kondensatoren 20 durch den Trockensubstanzgehalt (TS) unterschiedlich beeinflusst und/oder verändert. Die Fig. 6 zeigt den Kapazitätsverlauf des Kondensators 20, der durch die Sendeelektrode 6 und die Nahelektrode 7 ausgebildet ist. Die Fig. 7 zeigt den Kapazitätsverlauf des Kondensators, der durch die Sendeelektrode 6 und die Mittelelektrode 8 ausgebildet ist, für verschiedene Trockensubstanzgehälter 33, 34, 35 und 36. Die Fig. 8 zeigt den Kapazitätsverlauf des Kondensators, der durch die Sendeelektrode 6 und die Außenelektrode 9 ausgebildet ist.

Diese unterschiedlichen Einflüsse werden in der Auswertung der Signale bzw. Kapazitäten ausgenutzt. Je nach Abstand und Geometrie der Elektroden 5 haben die kapazitiven Sensoren 20 unterschiedliche Kennlinien. Die Kennlinien sind in unterschiedlichem Maße abhängig von den Messgrößen. Diese Eigenschaft wird ausgenutzt, um aus den Messwerten mittels einem Kennlinienfeld die geforderten Daten zu extrahieren. Aus einem kalibrierten Kennlinienfeld oder mehreren kalibrierten Kennlinienfeldern können aus diesen Messergebnissen die einzelnen Messgrößen ermittelt werden.

Die gemeinsame Sendeelektrode 6 wird beispielsweise mit einer Wechselspannung beaufschlagt. Die Gegenelektroden 7, 8, 9, 7a, 8a, 9a werden ausgewertet. Über den Transimpedanzverstärker 16 wird der Strom in eine Spannung gewandelt und mit der Detektorschaltung 17 ausgewertet. Diese Spannung wird über den AD-Wandler 19 digitalisiert. Die Messergebnisse für die einzelnen Eindringstufen werden aufgenommen und in die Steuerungs- und Regeleinrichtung 21 überführt. In dieser Steuerungs- und Regeleinrichtung 21 werden kalibrierte Kennlinien verwendet, um aus den Messwerten die die förderspezifischen Parameter und/oder gutspezifischen Parameter zu bestimmen. Dieses Kennlinienfeld kann in einem CEMOS-System integriert werden. Durch zusätzliche Messgrößen aus anderen Sensoren kann die Messung weiter optimiert werden, da beispielsweise eine Einflussgröße aus den Kennlinien reduziert werden kann.

Für die Bestimmung der Veränderung der Aufbereitung kann beispielsweise das Signal der Dichte verwendet werden. Unterschiedliche Aufbereitungsgrade am Corn-Cracker bzw. an der Konditioniereinrichtung 25 sorgen für feineres bzw. gröberes Häckselgut. Feineres Material sorgt für eine höhere Dichte im Auswurfkrümmer 3 und umgekehrt. Dementsprechend kann mit einem Dichtesignal eine relative Änderung der Gutaufbereitung (bei gleichbleibender Häcksellänge) erfasst werden. Durch die kontinuierliche Messung der Dichte wird so ein relativer Zusammenhang mit der Aufbereitung erfasst. Ändert sich der Grad der Aufbereitung, so verändert sich auch das Dichtesignal. Diese Erfassung ist eine Ausgangsbasis für eine adaptive Steuerung der Aufbereitungsaggregate. Nimmt die Dichte ab, kann von einer Verschlechterung der Aufbereitung ausgegangen werden und der Spaltabstand im Corn-Cracker bzw. in der Konditioniereinrichtung 25 nachgeregelt werden. Im Vergleich zu bildgebenden Verfahren wird beispielsweise keine absolute Erfassung der Aufbereitung angestrebt, sondern ein relativer Ansatz gewählt. Der Vorteil liegt in den deutlich geringeren Kosten und dem geringeren Verarbeitungsaufwand der Messsignale. Wie beschrieben, verändert eine Dichteänderung die Kapazität der Sensoren bzw. Kondensatoren 5. Die Dichteänderung verändert insbesondere die Kapazität der Kondensatoren 5 mit den Elektroden 6 und 8 und/oder 6 und 8a.

### Bezugszeichenliste

- 1: Feldhäcksler
- 2: Erntegut, Erntegutbestand
- 3: Auswurfkrümmer
- 3a: Oberseite
- 3b: Unterseite
- 4: Messvorrichtung
- 5: Elektrode
- 6: Sendeelektrode
- 7, 7a: Nahelektrode
- 8, 8a: Mittelelektrode
- 9, 9a: Außenelektrode
- 10: Längsrichtung
- 11: Querrichtung
- 12: Höhenrichtung
- 13: Wechselspannungserzeuger
- 14: Verstärkereinheit
- 15: Anschlussbauteil
- 16: elektrischer Verstärker
- 17: Detektor, insbesondere logarithmischer Detektor
- 18: Verstärkereinheit
- 19: Analog-Digital-Wandler
- 20: Kondensator
- 21: Steuerungs- und Regelungseinrichtung
- 22: Vorsatzgerät
- 23: Erntegutbearbeitungskanal
- 24: Häckseltrommel
- 25: Konditioniereinrichtung
- 26: Nachbeschleunigungstrommel
- 27: Längsabschnitt
- 27a: Längsabschnitt
- 28: Querabschnitt
- 28a: Querabschnitt
- 29: Förderrichtung
- 30: Erntegutstrom
- 31: Kapazität in pF
- 32: Ernteguthöhe in mm
- 33: Trockensubstanzgehalt 64,50 %
- 34: Trockensubstanzgehalt 67,35 %
- 35: Trockensubstanzgehalt 70,22 %
- 36: Trockensubstanzgehalt 73,88 %
- FR: Fahrtrichtung

## Patentansprüche

1. Selbstfahrender Feldhäcksler (1) zur Aufnahme und Behandlung von Erntegut (2),
- mit einem Auswurfkrümmer (3) zum Abtransport des Ernteguts (2),
- mit einer Messvorrichtung (4) zur Erfassung förderspezifischer Parameter und/oder gutspezifischer Parameter,
**dadurch gekennzeichnet,**
- **dass** die Messvorrichtung (4) mehrere voneinander beabstandete Elektroden (5) aufweist, die im Auswurfkrümmer (3) angeordnet sind,
- wobei diese im Auswurfkrümmer (3) angeordneten Elektroden (5) mehrere Kondensatoren (20) ausbilden,
- wobei durch Messungen der elektrischen Kapazitäten der mehreren Kondensatoren (20) die förderspezifischen Parameter und/oder gutspezifischen Parameter ermittelbar sind, wobei die Messvorrichtung (4) mehrere Elektroden (5) aufweist, die unterschiedliche Beabstandungen zueinander und/oder unterschiedliche Beabstandungen zu einer gemeinsamen Elektrode (5) aufweisen.

2. Selbstfahrender Feldhäcksler (1) nach Anspruch 1,
**dadurch gekennzeichnet,**
**dass** die Messvorrichtung (4) dazu ausgebildet ist, mittels der gemessenen Kapazitäten der Kondensatoren (20) eine Feuchtigkeitsmessung zur Ermittlung der Feuchtigkeit des Ernteguts (2) auszuführen.

3. Selbstfahrender Feldhäcksler (1) nach Anspruch 1 oder 2,
**dadurch gekennzeichnet,**
**dass** die Messvorrichtung (4) dazu ausgebildet ist, mittels der gemessenen Kapazitäten der Kondensatoren (20) eine Durchsatzmessung zur Ermittlung des Durchsatzes des Ernteguts (2) auszuführen.

4. Selbstfahrender Feldhäcksler (1) nach einem der vorhergehenden Ansprüche,
**dadurch gekennzeichnet,**
**dass** die Messvorrichtung (4) dazu ausgebildet ist, mittels der gemessenen Kapazitäten der Kondensatoren (20) eine Dichtemessung zur Ermittlung der Dichte des Ernteguts (2) auszuführen.

5. Selbstfahrender Feldhäcksler (1) nach einem der vorhergehenden Ansprüche,
**dadurch gekennzeichnet,**
**dass** die Messvorrichtung (4) dazu ausgebildet ist, mittels der gemessenen Kapazitäten aller Kondensatoren (20) eine Querverteilung des Ernteguts (2) zu ermitteln.

6. Selbstfahrender Feldhäcksler (1) nach einem der vorhergehenden Ansprüche,
**dadurch gekennzeichnet,**
**dass** jeder Kondensator (20) genau zwei Elektroden (5) aufweist, wobei diese Elektroden (5) nicht von einem anderen Kondensator (20) nutzbar sind.

7. Selbstfahrender Feldhäcksler (1) nach einem der Ansprüche 1 bis 5,
**dadurch gekennzeichnet,**
**dass** alle Kondensatoren (20) jeweils eine Elektrode (5) aufweisen, wobei diese Elektroden (5) jeweils mit einer gemeinsamen Elektrode (5), insbesondere mit einer gemeinsamen Sendeelektrode (5, 6), nutzbar sind.

8. Selbstfahrender Feldhäcksler (1) nach einem der vorhergehenden Ansprüche,
**dadurch gekennzeichnet,**
**dass** die Messvorrichtung (4) mehrere Elektroden (5) aufweist, die unterschiedliche geometrische Ausgestaltungen aufweisen.

9. Selbstfahrender Feldhäcksler (1) nach einem der vorhergehenden Ansprüche,
**dadurch gekennzeichnet,**
**dass** alle Elektroden (5) der Messvorrichtung (4) in einer Ebene angeordnet sind.

10. Selbstfahrender Feldhäcksler (1) nach einem der vorhergehenden Ansprüche,
**dadurch gekennzeichnet,**
**dass** einige, insbesondere alle, Elektroden (5) der Messvorrichtung (4) an einer Oberseite (3a) des Auswurfkrümmer (3) und/oder in einer Oberseite (3a) des Auswurfkrümmer (3) angeordnet sind.

11. Selbstfahrender Feldhäcksler (1) nach einem der vorhergehenden Ansprüche,
- dass die Messvorrichtung (10) wenigstens einen Wechselspannungserzeuger (13) zum Erzeugen einer Wechselspannung aufweist, und/oder
- dass die Messvorrichtung (10) wenigstens eine Verstärkereinheit (14, 18) zum Verstärken eines Signals aufweist, und/oder
- dass die Messvorrichtung (10) wenigstens einen elektrischen Verstärker (16) zur Umwandlung eines Eingangsstroms in eine proportionale Ausgangsspannung aufweist, und/oder
- dass die Messvorrichtung (10) wenigstens einen Detektor (17), insbesondere wenigstens einen logarithmischen Detektor, zur Umwandlung eines einer Wechselspannung in ein Leitungssignal aufweist, und/oder
- dass die Messvorrichtung (10) wenigstens einen Analog-Digital-Wandler (19) zur Umwandlung eines analogen Signals in ein digitales Signal aufweist.

12. Selbstfahrender Feldhäcksler (1) nach einem der vorhergehenden Ansprüche, **dadurch gekennzeichnet,**
- **dass** die Messvorrichtung (4) eine Steuerungs- und Regelungseinrichtung (21) aufweist, die dazu ausgebildet ist, die Kapazität der Kondensatoren (20) zu erfassen,
- wobei die Steuerungs- und Regelungseinrichtung (21) dazu ausgebildet ist, mittels der erfassten Kapazitäten die förderspezifischen Parameter und/oder gutspezifischen Parameter des Ernteguts (2) zu ermitteln,
- wobei die Steuerungs- und Regelungseinrichtung (21) dazu ausgebildet ist, Aktoren des Vorsatzgerätes (22) und/oder Aktoren des Erntegutbearbeitungskanals (23) anhand der ermittelten förderspezifischen Parameter und/oder gutspezifischen Parameter des Ernteguts (2) zu steuern.

13. Verfahren zum Steuern eines selbstfahrenden Feldhäckslers (1) nach einem der vorangehenden Ansprüche, mit den Schritten:
- Erfassen der Kapazität der Kondensatoren (20),
- Ermitteln der förderspezifischen Parameter und/oder gutspezifischen Parameter des Ernteguts (2) basierend auf den erfassten Kapazitäten, wobei die Messvorrichtung (4) mehrere Elektroden (5) aufweist, die unterschiedliche Beabstandungen zueinander und/oder unterschiedliche Beabstandungen zu einer gemeinsamen Elektrode (5) aufweisen.

14. Verfahren nach Anspruch 13,
**dadurch gekennzeichnet,**
- **dass** das Erfassen der Kapazität der Kondensatoren (20) zeitlich nacheinander erfolgt, und/oder
- **dass** das Erfassen der Kapazität der Kondensatoren (20) zeitgleich erfolgt.

## Claims

1. Self-propelled forage harvester (1) for picking up and treating crops (2),
- having a discharge chute (3) for transporting away the crop (2),
- having a measuring device (4) for detecting conveying-related parameters and/or product-related parameters,
**characterized**
- **in that** the measuring device (4) has a plurality of spaced-apart electrodes (5) which are arranged in the discharge chute (3),
- wherein these electrodes (5) arranged in the discharge chute (3) form a plurality of capacitors (20),
- wherein the delivery-related parameters and/or product-related parameters can be determined by measuring the electrical capacitances of the plurality of capacitors (20), wherein the measuring device (4) has a plurality of electrodes (5) which are at different distances from each other and/or at different distances from a common electrode (5).

2. Self-propelled forage harvester (1) according to Claim 1,
**characterized**
**in that** the measuring device (4) is designed to perform a moisture measurement for determining the moisture of the crop (2) by means of the measured capacitances of the capacitors (20).

3. Self-propelled forage harvester (1) according to Claim 1 or 2,
**characterized**
**in that** the measuring device (4) is designed to perform a throughput measurement for determining the throughput of the crop (2) by means of the measured capacitances of the capacitors (20).

4. Self-propelled forage harvester (1) according to any of the preceding claims,
**characterized**
**in that** the measuring device (4) is designed to perform a density measurement for determining the density of the crop (2) by means of the measured capacitances of the capacitors (20).

5. Self-propelled forage harvester (1) according to any of the preceding claims,
**characterized**
**in that** the measuring device (4) is designed to determine a lateral distribution of the crop (2) by means of the measured capacitances of all of the capacitors (20).

6. Self-propelled forage harvester (1) according to any of the preceding claims,
**characterized**
**in that** each capacitor (20) has exactly two electrodes (5), wherein these electrodes (5) cannot be used by another capacitor (20).

7. Self-propelled forage harvester (1) according to any of Claims 1 to 5,
**characterized**
**in that** all of the capacitors (20) each have an electrode (5), wherein these electrodes (5) can each be used with a common electrode (5), in particular with a common transmitting electrode (5, 6).

8. Self-propelled forage harvester (1) according to any of the preceding claims,
**characterized**
**in that** the measuring device (4) has a plurality of electrodes (5) which have different geometrical configurations.

9. Self-propelled forage harvester (1) according to any of the preceding claims,
**characterized**
**in that** all of the electrodes (5) of the measuring device (4) are arranged in one plane.

10. Self-propelled forage harvester (1) according to any of the preceding claims,
**characterized**
**in that** some, in particular all, of the electrodes (5) of the measuring device (4) are arranged on a top side (3a) of the discharge chute (3) and/or in a top side (3a) of the discharge chute (3).

11. Self-propelled forage harvester (1) according to any of the preceding claims,
- in that the measuring device (10) has at least one AC voltage generator (13) for generating an AC voltage, and/or
- in that the measuring device (10) has at least one amplifier unit (14, 18) for amplifying a signal, and/or
- in that the measuring device (10) has at least one electrical amplifier (16) for converting an input current into a proportional output voltage, and/or
- in that the measuring device (10) has at least one detector (17), in particular at least one logarithmic detector, for converting one of an AC voltage into a line signal, and/or
- in that the measuring device (10) has at least one analogue-to-digital converter (19) for converting an analogue signal into a digital signal.

12. Self-propelled forage harvester (1) according to any of the preceding claims,
**characterized**
- **in that** the measuring device (4) has an open-loop and closed-loop control device (21) which is designed to detect the capacitance of the capacitors (20),
- wherein the open-loop and closed-loop control device (21) is designed to determine the conveying-related parameters and/or product-related parameters of the crop (2) by means of the detected capacitances,
- wherein the open-loop and closed-loop control device (21) is designed to control actuators of the attachment (22) and/or actuators of the crop processing channel (23) based on the determined conveying-related parameters and/or product-related parameters of the crop (2).

13. Method for controlling a self-propelled forage harvester (1) according to any of the preceding claims, comprising the steps of:
- detecting the capacitance of capacitors (20),
- determining the conveying-related parameters and/or product-related parameters of the crop (2) on the basis of the detected capacitances, wherein the measuring device (4) comprises a plurality of electrodes (5) which are at different distances from each other and/or at different distances from a common electrode (5).

14. Method according to Claim 13,
**characterized**
- **in that** the capacitance of the capacitors (20) is detected in succession, and/or
- **in that** the capacitance of the capacitors (20) is detected at the same time.

## Revendications

1. Ensileuse (1) automotrice pour la récolte et le traitement de récoltes (2),
- avec une goulotte d'éjection (3) destinée à évacuer les récoltes (2),
- avec un dispositif de mesure (4) destiné à détecter des paramètres spécifiques au transport et/ou des paramètres spécifiques à la qualité,
**caractérisée en ce**
- **que** le dispositif de mesure (4) comporte plusieurs électrodes (5) espacées les unes des autres, qui sont disposées dans la goulotte d'éjection (3),
- lesdites électrodes (5) disposées dans la goulotte d'éjection (3) formant plusieurs condensateurs (20),
- les paramètres spécifiques au transport et/ou les paramètres spécifiques à la qualité pouvant être déterminés par des mesures des capacités électriques des plusieurs condensateurs (20), le dispositif de mesure (4) comportant plusieurs électrodes (5), qui comportent des distances différentes les unes par rapport aux autres et/ou des distances différentes par rapport à une électrode commune (5).

2. Ensileuse (1) automotrice selon la revendication 1, **caractérisée en ce**
**que** le dispositif de mesure (4) est formé pour exécuter une mesure d'humidité pour déterminer l'humidité des récoltes (2) au moyen des capacités mesurées des condensateurs (20).

3. Ensileuse (1) automotrice selon la revendication 1 ou 2,
**caractérisée en ce**
**que** le dispositif de mesure (4) est formé pour exécuter une mesure de débit pour déterminer le débit des récoltes (2) au moyen des capacités mesurées des condensateurs (20).

4. Ensileuse (1) automotrice selon l'une des revendications précédentes,
**caractérisée en ce**
**que** le dispositif de mesure (4) est formé pour exécuter une mesure de densité pour déterminer la densité des récoltes (2) au moyen des capacités mesurées des condensateurs (20).

5. Ensileuse (1) automotrice selon l'une des revendications précédentes,
**caractérisée en ce**
**que** le dispositif de mesure (4) est formé pour déterminer une répartition transversale des récoltes (2) au moyen des capacités mesurées de tous les condensateurs (20).

6. Ensileuse (1) automotrice selon l'une des revendications précédentes,
**caractérisée en ce**
**que** chaque condensateur (20) comporte exactement deux électrodes (5), lesdites électrodes (5) ne pouvant pas être utilisées par un autre condensateur (20).

7. Ensileuse (1) automotrice selon l'une des revendications 1 à 5,
**caractérisée en ce**
**que** tous les condensateurs (20) comportent chacun une électrode (5), lesdites électrodes (5) pouvant être utilisées chacune avec une électrode commune (5), en particulier avec une électrode d'émission commune (5, 6).

8. Ensileuse (1) automotrice selon l'une des revendications précédentes,
**caractérisée en ce**
**que** le dispositif de mesure (4) comporte plusieurs électrodes (5), qui présentent des configurations géométriques différentes.

9. Ensileuse (1) automotrice selon l'une des revendications précédentes,
**caractérisée en ce**
**que** toutes les électrodes (5) du dispositif de mesure (4) sont disposées dans un plan.

10. Ensileuse (1) automotrice selon l'une des revendications précédentes,
**caractérisée en ce**
**que** certaines, en particulier toutes les électrodes (5) du dispositif de mesure (4) sont disposées sur une face supérieure (3a) de la goulotte d'éjection (3) et/ou dans une face supérieure (3a) de la goulotte d'éjection (3).

11. Ensileuse (1) automotrice selon l'une des revendications précédentes,
- en ce que le dispositif de mesure (10) comporte au moins un générateur de tension alternative (13) pour produire une tension alternative, et/ou
- que le dispositif de mesure (10) comporte au moins une unité d'amplification (14, 18) destinée à amplifier un signal, et/ou
- que le dispositif de mesure (10) comporte au moins un amplificateur électrique (16) destiné à convertir un courant d'entrée en une tension de sortie proportionnelle, et/ou
- que le dispositif de mesure (10) comporte au moins un détecteur (17), en particulier au moins un détecteur logarithmique, destiné à convertir l'une d'une tension alternative en un signal de câble, et/ou
- que le dispositif de mesure (10) comporte au moins un convertisseur analogique-numérique (19) destiné à convertir un signal analogique en un signal numérique.

12. Ensileuse (1) automotrice selon l'une des revendications précédentes,
**caractérisée en ce**
- **que** le dispositif de mesure (4) comporte un système de commande et de régulation (21) qui est formé pour détecter la capacité des condensateurs (20),
- le système de commande et de régulation (21) étant formé pour déterminer les paramètres spécifiques au transport et/ou les paramètres spécifiques à la qualité des récoltes (2) au moyen des capacités détectées,
- le système de commande et de régulation (21) étant formé pour commander des actionneurs de l'accessoire (22) et/ou des actionneurs du canal de traitement de récolte (23) à l'aide des paramètres spécifiques au transport et/ou des paramètres spécifiques à la qualité des récoltes (2) déterminés.

13. Procédé de commande d'une ensileuse (1) automotrice selon l'une des revendications précédentes, avec les étapes :
- détection la capacité des condensateurs (20),
- détermination des paramètres spécifiques au transport et/ou des paramètres spécifiques à la qualité des récoltes (2) sur la base des capacités détectées, le dispositif de mesure (4) comportant plusieurs électrodes (5), qui présentent des distances différentes les unes par rapport aux autres et/ou des distances différentes par rapport à une électrode commune (5).

14. Procédé selon la revendication 13,
**caractérisé en ce**
- **que** la capacité des condensateurs (20) est détectée successivement chronologiquement, et/ou
- **que** la capacité des condensateurs (20) est détectée simultanément.
